# EUROPÄISCHE PATENTANMELDUNG

(11) **EP 0 696 587 A2**
(43) Veröffentlichungstag der Anmeldung: **14.02.1996**
(21) Anmeldenummer: 95112005.4
(22) Anmeldetag: 31.07.1995
(51) Int. Cl.: C07D 307/92

(54) **Verfahren zur stereoselektiven Herstellung von (-)3a,6,6,9a-Tetramethyl-perhydronaphtho[2,1-b]furan**

(30) Priorität: 08.08.1994 DE 4428002
(71) Anmelder: BASF AKTIENGESELLSCHAFT, D-67056 Ludwigshafen (DE)
(72) Erfinder: Hüllmann, Michael, Dr., D-64625 Bensheim (DE); Becker, Rainer, Dr., D-67098 Bad Dürkheim (DE); Hasel, Winnfried, Dr., D-67059 Ludwigshafen (DE); Jessel, Barbara, Dr., D-67067 Ludwigshafen (DE)

(57) **Zusammenfassung**

Verfahren zur stereoselektiven Herstellung von (-)3a,6,6,9-Tetramethyl-perhydronaphtho[2,1-b]furan der Formel Ia 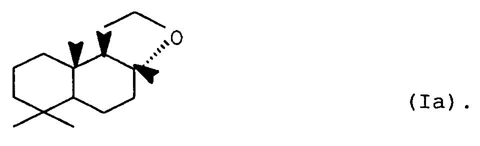
durch Dehydratisierung und Cyclisierung von Decahydro-2-hydroxy-2,5,5,8-tetramethyl-1-naphthalinethanol mittels festen sauren Katalysatoren, das dadurch gekennzeichnet ist, daß man das Decahydro-2-hydroxy-2,5,5,8-tetramethyl-1-naphthalinethanol in flüssiger Phase bei Temperaturen von 10 bis 50°C in Gegenwart von 10 % bis 100 Gew-%, bezogen auf das Diol, an einem kommerziell erhältlichen Montmorillonit-Kontakt mit hoher Ionenaustauschfähigkeit oder aber bei Temperaturen von 120 bis 140°C in Gegenwart von 10 bis 100 Gew.-% an einem kommerziell für die (präparative) Säulenchromatographie angebotenen aktiven sauren Aluminiumoxid umsetzt.

## Beschreibung

Die Erfindung betrifft ein Verfahren zur stereoselektiven Herstellung von (-) 3a,6,6,9-Tetramethyl-perhydronaphtho[2,1-b]furan der Formel Ia

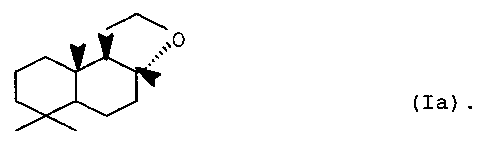

Diese Verbindung stellt einen seit Jahrzehnten in der Parfümerie verwendeten Ambra-Riechstoff (Ambrox® der Fa. Firmenich; Amboxan® der Fa. Henkel und Sylvamber® der Fa. BASF) dar.

Dieser Ambra-Riechstoff wurde inzwischen neben seinem Vorkommen in der Ambra-Tinktur des Pottwales ebenfalls in Muskateller-Salbei-Öl (Salvia Sclarea L.), im Labdanum-Öl (Cistus labdaniferus L.) sowie in Cypressen-Öl (Cupressus semperoirens L.) nachgewiesen (vgl. G. Ohloff in Fragrance Chemistry, Academic Press, 1982, S. 543). Er ruft den in Parfüm-Kompositionen begehrten "Ambra-Effekt" hervor, der auch in großer Verdünnung wirksam ist und sorgt darüber hinaus für eine ausgezeichnete Fixierung auch zarter, blumiger Düfte. Er wird hauptsächlich in teuren Parfümölen eingesetzt.

Die vier Diastereomeren dieses Ambra-Riechstoffs 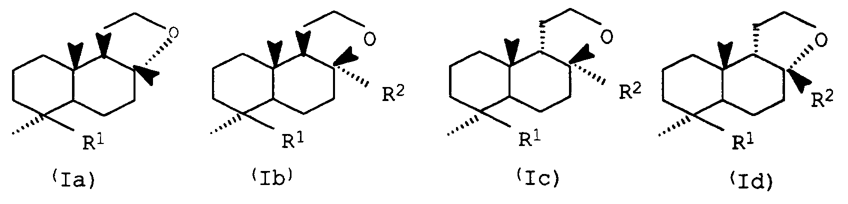
Ia-Id unterscheiden sich deutlich voneinander. So ist z.B. der Geruch des sogenannten "Isoambrox" (Ib) über hundertmal schwächer als der der Verbindung der Formel Ia (vgl. G. Ohloff et al. i. Helv. Chimica Acta, Vol. 68 (1985), s. 2022 bis 2029) und der Geruch des sogenannten "Norisoambrox" (Formel Ib mit R¹ = H und R² = CH₃) über 500 mal schwächer als der der Verbindung der Formel Ia.

Der Ambra-Riechstoff der Formel Ia bzw. die Diasteromeren-Gemische der Formeln Ia bis Id lassen sich sowohl partialsynthetisch aus Ambrein oder aus Diterpenen vom Labdan-Typ (z.B. Sclareol, Manool) als auch durch Totalsynthese herstellen (vgl. Übersicht von E.-J. Brunke, Dragoco-Report, 11/12 (1979) S. 276 ff).

Im technischen Maßstab wird der Ambra-Riechstoff hauptsächlich durch oxidativen Abbau (Chromsäure, Permanganat, Ozon) von Sclareol, einem Inhaltsstoff des Muskateller Salbei Concretes, hergestellt. Das als Abbau-Produkt erhaltene Sclareolid wird nach einer Lithiumalanat- oder Natriumboranat-Reduktion in ein 1,4-Diol, ("Ambroxdiol") übergeführt, das anschließend cyclisiert wird (vgl. G. Ohloff, Fortschr. Chem. Forsch. 12/2, (1969) 185 ff). 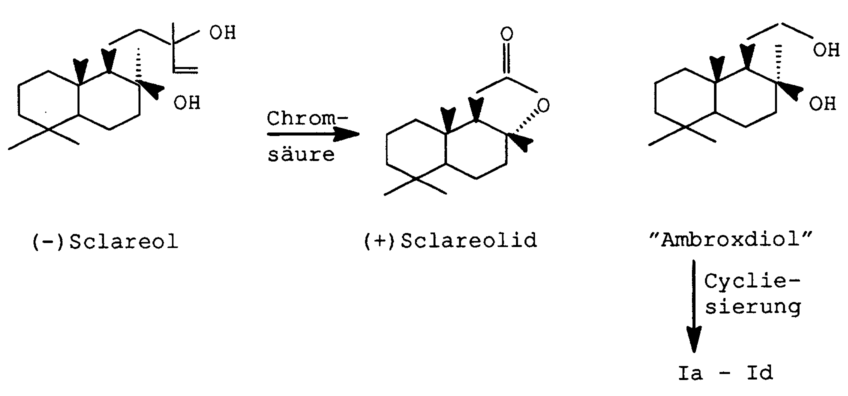
Aufgrund der Tatsache, daß unter den vier Diastereomeren die Verbindung der Formel Ia die größte olfaktorische Bedeutung besitzt, wurden in der Vergangenheit zahlreiche Verfahren entwickelt, die versuchten den letzten Schritt, die Cyclisierung, stereoselektiv durchzuführen.

So beschrieben V.E. Sibiertseva et al. in Maslo-Zhir, Prom.-st., 1979 (12), S. 25 bis 26, die Cyclisierung des "Ambroxdiols" in Gegenwart von p-Toluolsulfonsäure. Nachteilig an dem Verfahren ist die im sauren Medium nicht zu vermeidende Dehydratisierung der tertiären Hydroxygruppe, die zu Selektivitätsverlusten führt. Außerdem sind die angegebenen Ausbeuten von 55 bis 60 % bei einem technischen Verfahren unbefriedigend, insbesondere unter Berücksichtigung des teuren Edukts.

Die Verwendung von p-Toluolsulfonsäure wird auch in den Verfahren der russischen Patente SU 345 183 (von 1968), SU 910 561 (von 1980) sowie SU 529 166 (von 1975) beansprucht.

In dem Verfahren gemäß einem älteren Patent der Fa. Firmenich (DP 860 214 von 1949) wurden als Katalysatoren Naphthalinsulfonsaure (78 % Ausbeute) sowie Aluminiumoxid (50 % Ausbeute) beschrieben. Auch hierbei wurden die oben aufgeführten Nachteile, wie schlechte Selektivität, sowie unbefriedigende Ausbeuten beobachtet.

Gemäß dem Verfahren des spanischen Patents 432 815 (von 1976) wurde die Cyclisierung des "Ambroxdiols" mit Schwefelsäure durchgeführt; die hierbei erzielten Ausbeuten lagen bei nur 29 %.

R.C. Cambie et al. beschrieben in Aust. J. Chem. 24 (1971), S. 583 bis 591 sowie S. 2365 bis 2377, die Cyclisierung des "Ambroxdiols" mittels p-Toluolsulfonylchlorid in Pyridin mit 80 % Ausbeute. Hauptnachteile sind in diesem Falle der Einsatz des unangenehm riechenden Pyridins im Hinblick auf die Qualitätseinstellung des erhaltenen Riechstoffes sowie die relativ langen Reaktionszeiten der Umsetzung. Darüber hinaus ist bei der wäßrigen Aufarbeitung eine Pyridin-Rückführung durch dessen Wasserlöslichkeit unumgänglich, was zu einer Kostensteigerung führt.

Cambie verwendete bei einer zweiten Cyclisierungsmethode Schwefelsäure als Katalysator. Nachteilig sind hierbei die lange Reaktionszeit (>3 Tage) sowie die schlechten Ausbeuten (43 % der Theorie).

Auch bei dem vom Consertium Elektrochemie in der DOS 3 240 054 (von 1982) beschriebenen Cyclisierungsverfahren mit POCl₃ arbeitete man in wasserfreiem Pyridin, und man mußte daher die oben beschriebenen Nachteile in Kauf nehmen. Die Ausbeuten betrugen nur 65 % d. Theorie.

P.F. Vead and N.D. Unger beschrieben in "Synthesis", 1983, S. 816 bis 818 eine Cyclisierungsmethode, bei der als Reagens Trimethylchlorsilan in Dimethylsulfoxid (DMSO) eingesetzt wurde; die hierbei angegebenen Ausbeuten betragen 85 %. Wie aus dem hierin aufgeführten experimentellen Beispiel hervorgeht, stellt diese Methode nur ein Laborverfahren der (mg-Ansätze). Für die Technik von Nachteil ist der Einsatz des DMSO, das nach der wäßrigen Aufarbeitung aufgrund seiner Wasserlöslichkeit (Abwasserprobleme) aufwendig recyclisiert werden muß. Ein entscheidender Nachteil ist darüber hinaus die Notwendigkeit einer Reinigung des nach der Reaktion erhaltenen rohen Ambra-Reichstoffs auf eine sowohl chemisch als auch olfaktorisch akzeptierbare Qualität. Die chemische Reinigung erfolgte durch kostspielige Säulenchromatographie, wobei eine vollständige DMSO-Entfernung meist schwierig ist. Ein weiteres Problem ist das technisch häufig schwierige Handling von Trimethylchlorsilan, welches sehr korrosiv wirkt und außerdem toxikologisch bedenklich ist.

Die gleiche Cyclisierungsmethode wird auch im russischen Patent SU 988 817 (von 1980) beschrieben.

Weiterhin ist aus EP-A-204 009 ein Verfahren zur Herstellung von Amorox® bekannt, bei dem Sclareol auf biotechnischem Wege in das sogenannte "Ambroxdiol" überführt und dieses zum (-)3a,6,6,9a-Tetramethyl-perhydronaphtho[2,1-b]furan der Formel Ia cyclisiert wird. Die Cyclisierung wird mit einem Arylsulfonylchlorid in Gegenwart von sauren Verbindungen, wie HCl und sauren Ionenaustauschern oder in Gegenwart von Basen, wie Pyridin und NaOH vorgenommen. Bei der hier beschriebenen Cyclisierung in saurem Milieu werden schlechte Ausbeuten erzielt. Die Nachteile einer Cyclisierung in Gegenwart von organischen Basen, wie Pyridin im technischen Maßstab, wurden oben ausführlich erläutert.

Relativ gut gelingt die Cyclisierung mit Sulfonylchloriden in Gegenwart von Alkalihydroxiden. Nachteilig hierbei sind jedoch die relativ langen Reaktionszeiten; die noch ungenügenden Reinheiten der erhaltenen Verbindung der Formel Ia sowie die teilweise sehr hohe Reaktionstemperatur, die zu Selektivitätsverlusten führen kann.

Aus Chem. Abstr. 105, 134193 K (1986), ist weiterhin bekannt, daß bei der Verwendung von Weißerde, Tonerde oder Kieselerde, die mit 1 bis 20 Gew.-% Schwefelsäure, Phosphorsäure oder Polyphosphorsäure beladen sind, als Katalysatoren die theoretischen Ausbeuten an der Verbindung der Formel Ia auf 85 - 90,5 % gesteigert werden können. In 2 Beispielen wird die Reinheit der Verbindung der Formel Ia mit 97 % und 98 % angegeben.

Aus DE 39 12 318 ist weiterhin ein Verfahren zur Cyclisierung von "Ambroxdiol" in Gegenwart von säurebeladenen Aluminiumoxiden als Katalysatoren bekannt, welches durch folgende Merkmale charakterisiert ist:
1) Verwendung von 60 bis 80 Gew.-% Al₂O₃ bezogen auf 4
2) Salzsäurebeladung zwischen 0,4 und 0,6 Gew.-%
3) Schüttdichte 0,9 g/cm³
4) Kornbereich 0,05 bis 0,2 mm

Nachteilig hierbei sind jedoch, daß die Cyclisierung nicht in einem für die olfaktorischen Ansprüche ausreichendem Maße stereoselektiv ist und daß man ganz spezielle, mit Salzsäure vorbehandelte Aluminiumoxidkatalysatoren verwenden muß.

Es bestand somit die Aufgabe, ein hochselektives Cyclisierungsverfahren aufzufinden, das technisch auf einfache Weise durchführbar ist und das ohne die Nachteile der Verfahren des Standes der Technik zu einem olfaktorisch geeigneten Produkt führt.

Gegenstand der Erfindung ist ein Verfahren zur stereoselektiven Herstellung von (-)3a,6,6,9a-Tetramethyl-perhydronaphtho[2,1-b]-furan der Formel Ia 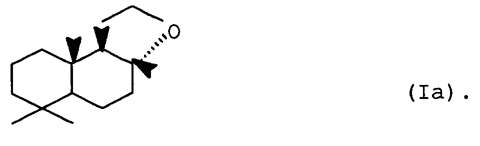
durch Dehydratisierung und Cyclisierung von Decahydro-2-hydroxy-2,5,5,8-tetramethyl-1-naphthalinethanol mittels festen sauren Katalysatoren, das dadurch gekennzeichnet ist, daß man das Decahydro-2-hydroxy-2,5,5,8-tetramethyl-1-naphthalinethanol in flüssiger Phase bei Temperaturen von 10 bis 50°C in Gegenwart von 10 % bis 100 Gew.-%, bezogen auf das Diol, an einem kommerziell erhältlichen Montmorillonit-Kontakt mit hoher Ionenaustauschfähigkeit oder aber bei Temperaturen von 120 bis 140°C in Gegenwart von 10 bis 100 Gew.-% an einem kommerziell für die (präparative) Säulenchromatographie angebotenen aktiven sauren Aluminiumoxid umsetzt.

Besonders reines Produkt erhält man, wenn man das Decahydro-2-hydroxy-2,5,5,8-tetramethyl-1-naphthalinethanol bei Raumtemperatur in Gegenwart von einem Montmorillonit-Kontakt umsetzt.

Mit besonderem Vorteil arbeitet man erfindungsgemäß so, daß man die Umsetzung des Decahydro-2-hydroxy-2,5,5,8-tetramethyl-1-naphthalinethanols in einem Lösungsmittel durchführt.

Das als Ausgangsverbindung verwendete Decahydro-2-hydroxy-2,5,5,8-tetramethyl-1-naphthalinethanol ist eine bekannte Verbindung und kann beispielsweise nach dem Verfahren der EP-A-204 009 hergestellt werden.

Unter einem kommerziell erhältlichen Montmorillonit-Kontakt verstehen wir erfindungsgemäß einen Montmorillonit der K-Serie der Firma Süd-Chemie, München, oder äquivalente Produkte anderer Katalysatorhersteller, insbesondere den sogenannten Katalysator KSF der Firma Süd-Chemie, der in Pulverform oder als Granulat (Körnung 30/60) im Handel ist. Mit Vorteil arbeitet man mit pulverförmigem KSF. Eine 10 %ige Suspension dieses Katalysators zeigt einen pH-Wert von 18, eine Cl⁻-Konzentration von nur 0,01 %, eine SO₄²⁻-Ionenkonzentration von kleiner als 0,01 %.

Dieser Katalysator ist leicht zu handhaben, zeigt keinerlei Korrosionswirkung und ist wirtschaftlich in seiner Anwendung. Besonders vorteilhaft ist, daß man bei der Verwendung von Montmorillonit einfach durch Rühren bei Raumtemperatur nahezu stereoselektiv reines (-)3a,6,6,9a-Tetramethyl-perhydronaphthol[2,1-b]furan der Formel Ia erhält, welches ausgezeichnete olfaktorische Eigenschaften aufweist. Die Menge an Montmorillonit-KSF-Katalysator beträgt 10 bis 100 Gew.-%, vorzugsweise 50 bis 80 Gew.-%, bezogen auf das eingesetzte Ziel.

Ein bezüglich der erzielbaren Ausbeuten sowie der sehr guten olfaktorischen Eigenschaften gleichwertiger Cyclisierungskatalysator ist kommerziell für die präparative Säulenchromatographie im Handel befindliches saures Aluminiumoxid, insbesondere das bei Firma E. Merck, Darmstadt erhältliche Aluminiumoxid 90, aktiv, sauer (Aktivitätsstufe I) für die Säulenchromatographie oder äquivalente Produkte anderer Firmen. Bei Verwendung des kommerziellen Alumniumoxid-Katalysators muß man zwar Temperaturen von 120 bis 140°C anwenden, jedoch wird hierdurch in Gegenwart dieses Katalysators die gewünschte Qualität des Produkts nicht beeinträchtigt. Die Reaktionszeiten betragen bei dieser Variante des erfindungsgemäßen Verfahrens nur etwa 5 bis 10, vorzugsweise 6 bis 8 Stunden.

Die erfindungsgemäße Umsetzung kann ohne Mitverwendung von Lösungsmitteln durchgeführt werden. Vorzugsweise arbeitet man jedoch in Lösungsmitteln. Als geeignete Lösungsmittel seien genannt: Kohlenwasserstoffe, wie Toluol oder Xylol; Alkohole, wie Methanol oder Ethanol oder Ether wie Methyl-tert.-butylether, Diethylether oder Tetrahydrofuran.

Als besonders geeignet erwiesen sich aromatische Kohlenwasserstoffe, wie Toluol oder Xylol, oder aber Gemische derselben. Die Lösungsmittel können auf relativ einfache Weise von dem Wertprodukt getrennt und so wieder in den Prozeß zurückgeführt werden.

Mit Hilfe des erfindungsgemäßen Verfahrens ist es möglich, Decahydro-2-hydroxy-2,5,5,8-tetramethyl-1-naphthalin-ethanol auf sehr einfache und trotzdem kostengünstige und umweltschonende Weise in sehr guten Ausbeuten stereoselektiv in das als Duftstoff begehrte (-)3a,6,6,9a-Tetramethyl-perhydronaphtho[2,1-b]furan zu überführen.

### Beispiel 1

5 g Decahydro-2-hydroxy-2,5,5,8-tetramethyl-1-naphthalin-ethanol (Ambroxdiol) und 5 g Montmorillonit KSF wurden in 100 ml Toluol suspendiert und die Suspension 24 Stunden (h) bei Raumtemperatur (RT) gerührt. Anschließend wurde der Katalysator abfiltriert, das Lösungsmittel abdestilliert und das Reaktionsprodukt gaschromatographisch untersucht.

Folgendes Ergebnis wurde erzielt:

| | |
|---|---|
| a) Umsatz | >99,9 % |
| b) Ausbeute an Ia | 95,9 % |
| c) Ausbeute an Isomeren von Ia | 4,0 % |
| d) Enthaltene Nebenprodukte | Σ0,1 % |

Alle %-Angaben sind GC-Flächenprozente.

### Beispiel 2

1200 g reines Ambroxdiol wurden zusammen mit 1200 g Aluminiumoxid 90 aktiv sauer (Aktivitätsstufe I) in 3000 ml Toluol 6 h unter Rückfluß zum Sieden erhitzt und dabei gebildetes Wasser mittels eines Wasserabscheiders abgetrennt. Zur Aufarbeitung wurde die Lösung heiß (100°C) abgesaugt und dreimal mit heißem Toluol gewaschen. Die vereinigten Filtrate wurden unter reduziertem Druck bei 60 bis 85°C eingeengt und das Reaktionsprodukt gaschromatographisch untersucht.

Folgendes Ergebnis wurde erzielt:

| | |
|---|---|
| a) Umsatz: | 99,5 % |
| b) Ausbeute an Ia: | 98,4 % |
| c) Ausbeute an Isomeren von Ia: | 0,18 % |
| | 1,03 % |
| | 0,27 % |
| d) Enthaltene Nebenprodukte: | 0,11 % |

Alle %-Angaben sind GC-Flächenprozente.

## Patentansprüche

1. Verfahren zur stereoselektiven Herstellung von (-)3a,6,6,9-Tetramethyl-perhydronaphtho[2,1-b]furan der Formel Ia 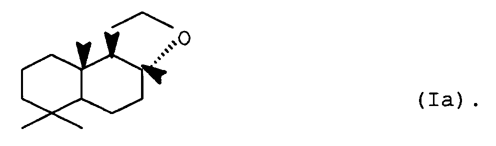 durch Dehydratisierung und Cyclisierung von Decahydro-2-hydroxy-2,5,5,8-tetramethyl-1-naphthalinethanol mittels festen sauren Katalysatoren, dadurch gekennzeichnet, daß man das Decahydro-2-hydroxy-2,5,5,8-tetramethyl-1-naphthalinethanol in flüssiger Phase bei Temperaturen von 10 bis 50°C in Gegenwart von 10 % bis 100 Gew-%, bezogen auf das Diol, an einem kommerziell erhältlichen Montmorillonit-Kontakt mit hoher Ionenaustauschfähigkeit oder aber bei Temperaturen von 120 bis 140°C in Gegenwart von 10 bis 100 Gew.-% an einem kommerziell für die (präparative) Säulenchromatographie angebotenen aktiven sauren Aluminiumoxid umsetzt.

2. Verfahren nach Anspruch 1, dadurch gekennzeichnet, daß man das Decahydro-2-hydroxy-2,5,5,8-tetramethyl-1-naphthalinethanol bei Raumtemperatur in Gegenwart von einem Montmorillonit-Kontakt umsetzt.

3. Verfahren nach Anspruch 1, dadurch gekennzeichnet, daß man die Umsetzung des Decahydro-2-hydroxy-2,5,5,8-tetramethyl-1-naphthalinethanols in einem Lösungsmittel durchführt.

4. Verfahren nach Anspruch 1, dadurch gekennzeichnet, daß man die Umsetzung des Decahydro-2-hydroxy-2,5,5,8-tetramethyl-1-naphthalinethanols in Toluol oder Xylol als Lösungsmittel durchführt.

5. Verfahren gemäß Anspruch 1, dadurch gekennzeichnet, daß man die Umsetzung des Decahydro-2-hydroxy-2,5,5,8-tetramethyl-1-naphthalinethanols in Gegenwart von Katalysatoren der K-Serie der Firma Süd-Chemie, München, als Montmorillonit-Kontakt mit hoher Ionenaustauschfähigkeit umsetzt.

6. Verfahren gemäß Anspruch 1, dadurch gekennzeichnet, daß man die Umsetzung des Decahydro-2-hydroxy-2,5,5,8-tetramethyl-1-naphthalinethanols in Gegenwart von einem Montmorillonit KSF der Firma Süd-Chemie, Münschen, als Montmorillonit-Kontakt durchführt.

7. Verfahren gemäß Anspruch 1, dadurch gekennzeichnet, daß man die Umsetzung des Decahydro-2-hydroxy-2,5,5,8-tetramethyl-1-naphthalinethanols in Gegenwart von Aluminiumoxid 90 aktiv sauer (Aktivitätsstufe I) für die Säulenchromatographie der Firma E. Merck, Darmstadt, durchführt.

8. Verfahren gemäß Anspruch 1, dadurch gekennzeichnet, daß man das Decahydro-2-hydroxy-2,5,5,8-tetramethyl-1-naphthalinethanol ggf. in einem Lösungsmittel und in Gegenwart von einem Montmorillonit-KSF-Kontakt der Firma Süd-Chemie, München, 10 bis 24 Stunden bei Raumtemperatur rührt.

9. Verfahren gemäß Anspruch 1, dadurch gekennzeichnet, daß man das Decahydro-2-hydroxy-2,5,5,8-tetramethyl-1-naphthalinethanol ggf. in einem Lösungsmittel und in Gegenwart von Aluminiumoxid 90 aktiv sauer (Aktivitätsstufe I) für die Säulenchromatographie der Firma Merck, Darmstadt, für 5 bis 10 Stunden auf Temperaturen von 120 bis 140°C erhitzt.
